(19) Europäisches Patentamt
European Patent Office
Office européen des brevets

(11) Numéro de publication: **0 013 995**
**B1**

(12) # FASCICULE DE BREVET EUROPEEN

(45) Date de publication du fascicule du brevet: **29.12.82**

(21) Numéro de dépôt: **80100397.1**

(22) Date de dépôt: **25.01.80**

(51) Int. Cl.³: **C 07 C 45/51,**
C 07 C 49/587
//C07C143/825

(54) **Procédé pour la préparation de cétones macrocycliques acétyleniques.**

(30) Priorité: **26.01.79 CH 796/79**

(43) Date de publication de la demande:
**06.08.80 Bulletin 80/16**

(45) Mention de la délivrance du brevet:
**29.12.82 Bulletin 82/52**

(84) Etats contractants désignés:
**CH DE FR GB IT NL SE**

(56) Documents cités:
**JOURNAL OF ORGANIC CHEMISTRY, vol. 39, no. 24, 1974, pages 3504—3506**
**G. ROSINI: "Reaction of p-Toluene-sulfonylhydrazones with N-Bromosuccinimmide in Methanol. Regeneration of Carbonyl Compounds"**

**Le dossier contient des informations techniques présentées postérieurement au dépôt de la demande et ne figurant pas dans le présent fascicule.**

(73) Titulaire: **FIRMENICH SA**
**Case postale 239**
**CH-1211 Genève 8 (CH)**

(72) Inventeur: **Fehr, Charles**
**16 bis, ch. des Genêts**
**CH-1202 Geneve (CH)**
Inventeur: **Ohloff, Günther**
**13, ch. de la Chapelle**
**CH-1233 Bernex/Ge (CH)**
Inventeur: **Büchi, George H.**
**100 Memorial Drive**
**Cambridge, Mass. 02142 (US)**

(74) Mandataire: **Schmied-Kowarzik, Volker, Dr. et al, Patentanwälte Dipl.-Ing. P. Wirth Dr. V. Schmied-Kowarzik Dipl.-Ing. G. Dannenberg Dr. P. Weinhold Dr. D. Gudel Dipl.-Ing. S. Schubert Siegfriedstrasse 8 D-8000 München 40 (DE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 013 995 B1

(56) Documents cités


**CHEMICAL ABSTRACTS, vol. 89, no. 13, 25 Septembre 1978, page 817, no. 108366a Columbus, Ohio, U.S.A.**

**HELVETICA CHIMICA ACTA, vol. 54, Fasc. 7, no. 191, 1971, pages 1789—1796 Basel, CH. G. OHLOFF et al.: ""Nichtcarbenoide" Zwischenstufen bei der Fragmentierung von CC-Cyclopropyltosylhydrazonen"**

Procédé pour la préparation de cétones macrocycliques acétyléniques

L'invention a pour objet un procédé pour la préparation d'un composé cétonique acétylénique de formule

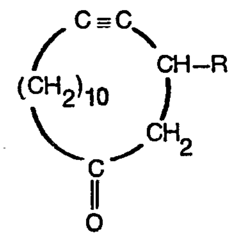

(I)

dans laquelle le symbole R représente un radical alkyle contenant de 1 à 3 atomes de carbone ou un atome d'hydrogène,
caractérisé en ce que
a) on traite une hydrazone de formule

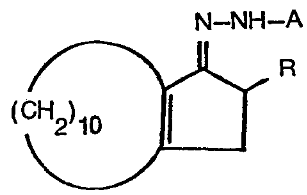

(II)

dans laquelle le symbole R est défini comme indiqué ci-dessus et A sert à désigner un radical sulfonyle de formule

$$R^1SO_2—$$ (III)

dans laquelle $R^1$ représente un radical aryle, au moyen d'un réactif halogénant choisi parmi les composés ci-après:

a. brome
b. chlore,
c. iode,
d. 1,3-dibromo-5,5-diméthyl-hydantoïne,
e. N-bromosuccinimide,
f. N-iodosuccinimide et
g. N-chlorosuccinimide

en présence d'alcool isopropylique, d'alcool butylique secondaire ou d'éthylène glycol et à une température comprise entre −25° et +25°C;
b) on réduit l'excès de réactif halogénant et
c) on chauffe enfin le produit de réaction en milieu aqueux, à une température comprise entre 20 et 60°C.

Parmi les composés possédant un caractère odorant musqué figurent notamment la cyclopentadécanone et son homologue supérieur, la 3-méthyl-cyclopentadécanone, mieux connus dans l'art sous la dénomination d'EXALTONE®, respectivement de muscone.

A ce jour, fort nombreuses ont été les méthodes de synthèses proposées pour leur préparation et certaines, parmi celles-ci, ont été développées à l'échelle industrielle [voir à ce sujet: Cosmetics and Perfumery, *88*, 67 (1973)]. Malgré cela, ces produits restent d'un accès difficile et à cause de leur prix élevé ont trouvé une utilisation relativement limitée.

Parmi les procédés de fabrication industrielle connus figure celui illustré à l'aide du schéma réactionnel suivant:

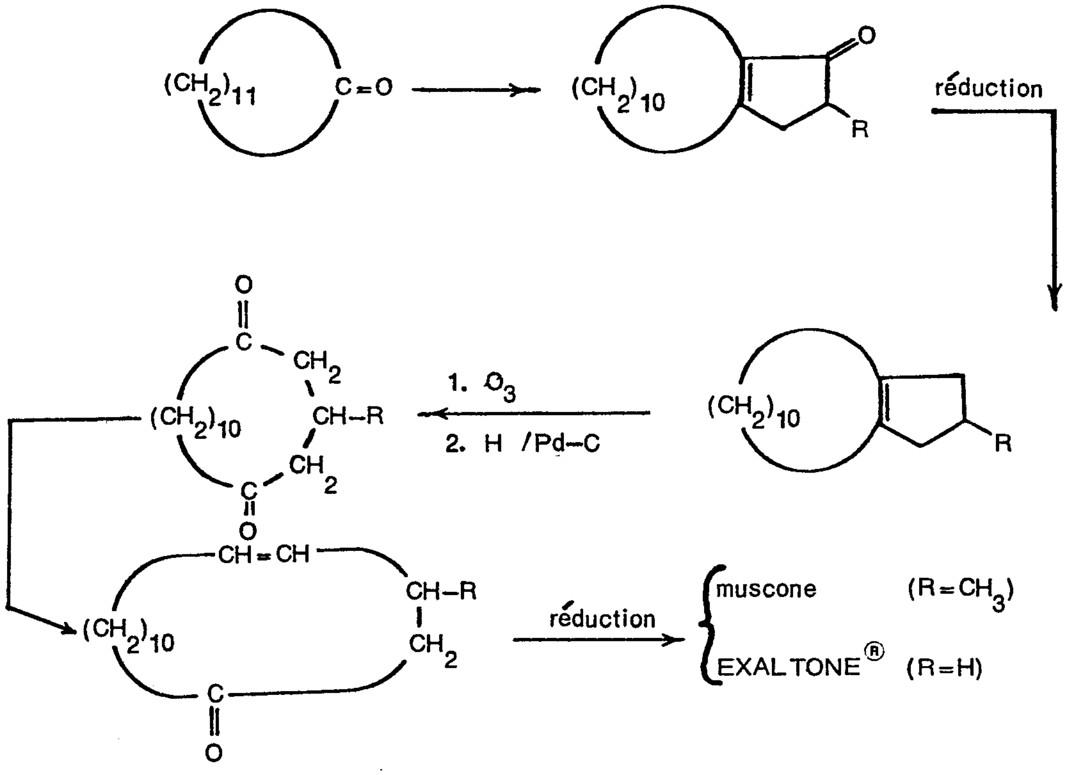


référence: Helv. Chim. Acta. *50*, 705 (1967)

ainsi que la variante ci-après:

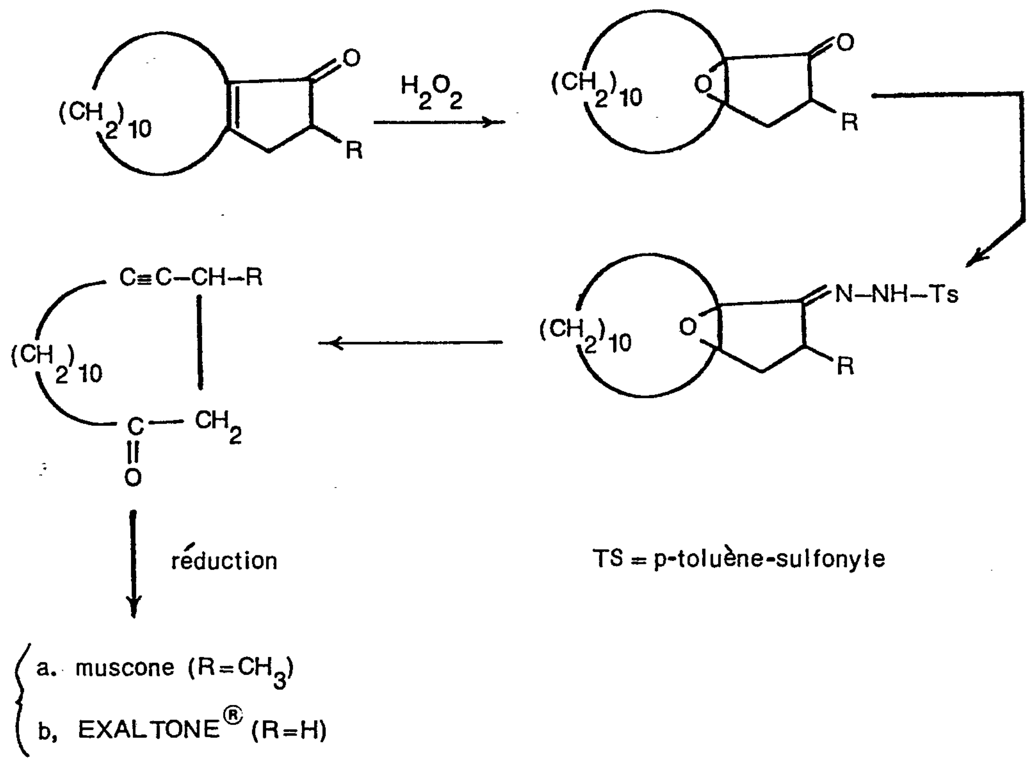


référence: Helv. Chim. Acta, *50*, 708 (1967)

Comme le montre le schéma réactionnel ci-dessus, les composés acétyléniques de formule (I) s'utilisent à titre de produits intermédiaires dans la préparation desdites cétones macrocycliques à odeur musouée.

La demande de brevet japonais publiée no. 53-34750 décrit un procédé pour la préparation de la cétone acétylénique de formule (I) dans laquelle le symbole R représente un atome d'hydrogène. Cette dernière est obtenue à partir de l'hydrazone de formule (II) correspondante après traitement au moyen d'acide hypochloreux ou de l'un de ses sels et en présence d'un sel de mercure ou d'argent ou d'acide formique.

Dans J. Org. Chemistry *39* 3504 (1974), il est fait mention de l'action, en présence de méthanol, de N-bromosuccinimide sur certaines p-toluènesulfonylhydrazones: le procédé décrit conduit à la régéneration de la cetone correspondants.

Grâce au procédé de l'invention, il est désormais possible d'obtenir les produits intermédiaires de formule (I) avec des rendements supérieurs à ceux des méthodes décrites ci-dessus: les cétones macrocycliques sus-mentionnées peuvent être obtenues par conséquent à des conditions plus avantageuses du point de vue économique.

Les hydrazones de formule (II), utilisées comme produits de départ dans le procédé de l'invention, sont des composés pouvant être aisément obtenus par des méthodes usuelles à partir des cétones correspondantes, dont la préparation est notamment décrite dans la DE—AS 16 68 054 et la DE—OS 26 30 428.

Comme hydrazones de formule (II), on utilise selon l'invention des dérivés sulfonylés, en particulier arylsulfonylés. C'est ainsi que le groupe sulfonyle peut représenter de Préference un radical de benzène substitué, comme p-toluènesulfonyle, p-nitrobenzènesulfonyle ou 2,4-dinitrobenzènesulfonyle par exemple.

La réaction peut s'effectuer en mettant en suspension l'hydrazone de départ soit directement dans l'alcool choisi, soit dans un mélange dudit alcool et un solvant auxiliaire, tel un éther comme le tétrahydrofuranne, le dioxanne ou l'éthylène-glycol diméthyl éther, l'acétone, ou encore tout mélange desdits solvants.

Comme indiqué plus haut, ladite réaction peut s'effectuer à une température comprise entre environ −25 et +25°C; de préférence on opère à environ −10°C. Il a été remarqué qu'à cette température les rendements en produit final étaient les plus élevés. A des températures au-delà de la limite supérieure indiquée, on a observé, par contre, la formation de produits secondaires indésirables.

Les cétones acétyléniques de formule (I) obtenues conformément au procédé de l'invention, sont accompagnées de quantités variables de cétones bicycliques correspondant aux hydrazones de départ utilisées. Il s'est avéré que la proportion desdites cétones acétyléniques dans les mélanges obtenus dépend en particulier de la nature de l'alcool aliphatique utilisé: par exemple, l'alcool isopropylique permet l'obtention de mélanges contenant presque exclusivement la cétone acétylénique, lorsque le procédé était appliqué à la bicyclo[10.3.0]pentadéc-[1(12)]ène-13-one p-toluène-sulfonyl-hydrazone en vue de la préparation de la cyclopentadéc-1-yne-5-one, tandis que l'éthylène-glycol permet l'obtention de mélanges ayant une teneur élevés en cétone acétylénique désirée, lorsque l'on applique le procédé à la 14-méthyl-bicyclo[10.3.0]pentadéc[1(12)]-ène-13-one p-toluènesulfonylhydrazone pour la préparation de la 3-méthyl-cyclopentadéc-1-yne-5-one.

Les temps de réaction sont très courts, de l'ordre de quelques minutes; le cours de la réaction est arrêté par l'adjonction au mélange réactionnel d'un réactif apte à réduire l'excès du réactif halogénant employé. A cet effet des sels de métaux alcalins du type sulfite, bisulfite et thiosulfate de sodium ou potassium en solution aqueuse sont utilisés de préférence. Après l'addition du réducteur, le mélange réactionnel est généralement chauffé à une température comprise entre environ 20 et 60°C. L'addition même du réactif réducteur au mélange de réaction est exothermique et, parfois, un chauffage extérieur se révèle superflu. Enfin, la séparation du produit désiré du mélange obtenu peut être effectuée au moyen des techniques habituelles, par exemple par distillation fractionnée.

La transformation des cétones acétyléniques obtenues par le procéde de l'invention en leur dérivés cétoniques saturés correspondants, EXALTONE® et muscone, peut s'effectuer conformément à des méthodes connues, notamment par hydrogénation catalytique [voir également DE—OS 26 30 428, p. 6 en particulier].

L'invention est illustrée d'une manière plus détaillée par les exemples suivants (températures en degrés centrigrades).

## Exemple 1

*Cyclopentadec-1-yne-5-one*

A une suspension de 3,88 g (10 mMole) de bicyclo[10.3.0]pentadéc-[1(12)]-éne-13-one p-toluènesulfonylhydrazone dans un mélange 1:1 (volume) d'acétone et alcool isopropylique (80 ml) on a ajouté à −15° sous vigoureuse agitation 3,15 g (11 mMole) de 1,3-dibromo-5,5-diméthyl-hydantoïne. Un fort dégagement de gaz a été observé pendant que le mélange se colorait en rouge et la température augmentait à −5°. Après trois minutes, le déroulement de la réaction a été arrêté par l'addition de 7,5 ml d'une solution aqueuse 2,75 M de bisulfite de sodium, puis 50 ml d'eau ont été ajoutés au mélange turbide jaunâtre résultant. Après avoir été maintenu pendant 30 min sous agitation à 50—55°C et refroidi par la suite, le mélange a été traité avec 100 ml d'éther de pétrole (Eb. 80—100°) et 70 g d'une solution aqueuse à 50% de NaOH. Une fois séparée, la phase aqueuse a été extraite trois fois avec de l'éther de pétrole et les extraits organiques combinés ont été soumis aux

traitements habituels de lavage avec eau, avec une solution saline saturée et séchés sur du sulfate de sodium.

L'évaporation des parties volatiles a permis d'obtenir 2,59 g d'une huile jaune-rouge constituée par un mélange 9:1 de cyclopentadéc-1-yne-5-one et bicyclo[10.3.0] pentadéc-[1(12)]-éne-13-one.

Le produit désiré a été obtenu par purification à l'aide d'une distillation au moyen d'un appareil à boules (140° temp. du bain/0,05 Torr.). On a pu ainsi obtenir 1,412 g (rend. 64%) du produit désiré ayant une pureté d'environ 90%, présentant des caractères analytiques identiques à ceux d'un échantillon prépare suivant une méthode connue [Helv. Chim. Acta, *54*, 2896 (1971)].

## Exemple 2

*Cyclopentadéc-1-yne-5-one*

En remplaçant dans le procédé décrit à l'Exemple 1, la 1,3-dibromo-5,5-diméthyl-hydantoïne par une quantité équivalente (21 mMole) de N-bromosuccinimide, on a obtenu des résultats presque identiques.

## Exemple 3

*3-Méthyl-cyclopentadéc-1-yne-5-one*

20,1 g (50 mMole) de 14 - méthyl - bicyclo[10.3.0]pentadéc - [1(12)] - ène - 13 - one p-toluènesulfonylhydrazone ont été dissous dans 500 ml d'un mélange 2:2:1 d'acétone, tétrahydrofuranne et éthylène-glycol et 20,0 g (112 mMole) de N-bromo-succinimide ont été ensuite ajoutés à la solution résultante refroidie à —15°. Un fort dégagement de gaz a été observé pendant que le mélange se colorait en rouge et la température augmentait à —6° environ en l'espace de 90 secondes.

Le déroulement de la réaction a été arrêté après 3 minutes par l'addition de 50 ml d'une solution aqueuse 2,75 M de bisulfite de sodium, puis 200 ml d'eau ont été ajoutés au mélange réactionnel. Après avoir été maintenu pendant 30 minutes à 40° le mélange a été traité comme indiqué à l'Exemple 1 pour fournir 13,8 g d'un mélange 5:1 de 3-méthyl-cyclopentadéc-1-yne-5-one et 14-méthyl-bicyclo[10-3-0]pentadéc-[1(12)]-ene-13-one.

6,9 g de ce mélange ont été distillés présence de 3 g de carbonate de potassium au moyen d'un appareil à boules (160°/0,1 Torr) pour donner 3,16 (13,5 mMole: rend. 54%) d'un mélange 85:15 de la cétone désirée et 14-méthylbicyclo[10.3.0]pentadéc-[1(12)]-ène-13-one. Une purification ultérieure a été effectuée au moyen d'une chromatographie sur colonne (gel de silice; éluant; cyclohexane/acétate d'éthyle 19/1) pour fournir la 3-méthyl-cyclopenta-déc-1-yne-5-one pure dont les caractères analytiques étaient en accord avec ceux d'un échantillon préparé conformément à une méthode décrite [Helv. Chim. Acta, *54*, 2896 (1971)].

## Exemple 4

*3-Méthyl-cyclopentadéc-1-yne-5-one*

En remplaçant dans le procédé décrit à l'Exemple 3, le N-bromosuccinimide (2,14 équivalents) par une quantité égale à 1,1 équivalents de 1,3-dibromo-5,5-diméthyl-hydantoïne, on a obtenu des résultats analogues.

Les dérivés p-toluènesulfonyl-hydrazone de la bicyclo[10.3.0]pentadéc-[1(12)]ène-13-one, respectivement 14-méthyl-bicyclo[10.3.0]pentadéc-[1(12)]-ène-13-one, utilisés comme produits de départ dans le procédé tel que décrit aux Exemples 1 à 4 peuvent être préparés ainsi:

Bicyclo[10.3.0]pentadéc-[1(12)]-ène-13-one p-toluènesulfonyl-hydrazone

Une suspension de 220 g (1 Mole) de bicyclo[10.3.0]-pentadéc-[1(12)]-ène-13-one, 205 g (1,1 Mole) de p-toluène-sulfonyl-hydrazine et 1000 ml d'éthanol à 95% a été chauffée dans un réacteur muni d'un système latéral de distillation équipé d'une colonne de type Vigreux (20 cm). La température du mélange a été maintenue de façon à permettre une distillation lente du solvant présent. Des quantités supplémentaires (1500 ml) d'éthanol ont été ajoutées au cours de cette évaporation. Après 7 h, le mélange de réaction a été refroidi et 100 ml d'eau y ont été ajoutés, puis le tout a été maintenu à 5° pendant une nuit au cours de laquelle l'hydrazone désirée cristallisait.

Une fois séparés par filtration, ce produit cristallin a été lavé avec 3 portions de 400 ml chacune d'un mélange 4:1 d'éthanol et eau, pour fournir enfin 346,8 g du produit désiré ayant F. 150,5—153°. Une autre portion contenant 15,8 g de cette même hydrazone a été séparés des eaux-mères par refroidissement prolongé de celles-ci à environ 5°.

Un échantillon analytique du produit obtenu a été purifié par recristallisation dans l'éthanol à 95%:

| | |
|---|---|
| F.(décomp.): | 164—167°; |
| IR($COCl_3$): | 3200, 3030—2750, 1615, 1600, 1460, 1440, 1395, 1325, 1180, 1160, 1085, 1015 $cm^{-1}$; |
| RMN($CDCl_3$): | 7,90 (2H); 7,77 (1H, s); 7,26 (2H); 2,40 (3H, s); 2,5—2,0 (8H, m); 1,9—1,1 (16H, m) $\delta$ ppm; |

SM: m/e: 28 (20), 32 (99), 41 (33), 43 (22), 55 (23), 57 (24), 67 (13), 68 (11), 69 (100), 70 (10), 71 (15), 81 (55), 82 (11), 83 (13), 93 (13), 95 (19), 109 (12), 121 (10), 137 (11).

14-Méthyl-bicyclo[10.3.0]pentadéc-[1(12)]-ène-13-one p-toluènesulfonyl-hydrazone

Ce composé a été obtenu opérant de façon tout à fait analogue à celle décrite ci-dessus, et en remplaçant la bicyclo[10.3.0]pentadéc-[1(12)]ène-13-one par la 14-méthyl-bicyclo[10.3.0]cyclopentadéc-[1(12)]-ène-13-one. Temps de chauffage: 24 h (rend. 66,5%)

F. (décomp.): 134—140°
IR($CDCl_3$): 3230, 3030—2800, 1620, 1600, 1465, 1445, 1395, 1370, 1330, 1160, 1090, 1015 $cm^{-1}$;
RMN($CDCl_3$): 7,87 (2H); 7,62 (1H); 7,26 (2H); 2,80 (1H); 2,40 (3H, s); 2,6—1,9 (6H, m); 1,9—1,0 (19H, m) $\delta$ ppm;
SM: m/e: 27 (18), 28 (100), 31 (38), 32 (37), 39 (20), 41 (26), 43 (27), 44 (93), 45 (17), 48 (12), 55 (22), 64 (27), 65 (13), 67 (10), 77 (11), 79 (15), 81 (12), 91 (78), 92 (50), 93 (18), 94 (34), 105 (16), 106 (12), 107 (21), 119 (14).

## Revendications

1. Procédé pour la préparation d'un composé cétonique acétylénique de formule

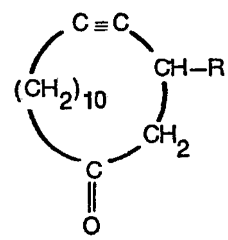

(I)

dans laquelle le symbole R représente un radical alkyle contenant de 1 à 3 atomes de carbone ou un atome d'hydrogène, caractérisé en ce que
a) on traite une hydrazone de formule

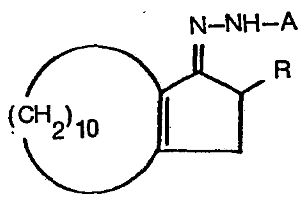

(II)

dans laquelle le symbole R est défini comme indiqué ci-dessus et A sert à désigner un radical sulfonyle de formule

$$R^1SO_2—$$ (III)

dans laquelle $R^1$ représente un radical aryle, au moyen d'un réactif halogénant choisi parmi les composés ci-après:

a. brome,
b. chlore,
c. iode,
d. 1,3-dibromo-5,5-diméthyl-hydantoïne,
e. N-bromosuccinimide,
f. N-iodosuccinimide et
g. N-chlorosuccinimide

en présence d'alcool isopropylique, d'alcool butylique secondaire ou d'éthylène glycol et à une température comprise entre −25° et +25°C;
b) on réduit l'excès de réactif halogénant et
c) on chauffe enfin le produit de réaction en milieu aqueux, à une température comprise entre 20 et 60°C.

2. Procédé selon la revendication 1, caractérisé en ce que l'hydrazone de formule (II) est la bicyclo[10.3.0]pentadéc-[1(12)]-ène-13-one p-toluènesulfonylhydrazone et le composé cétonique acétylénique de formule (I) obtenu est la cyclopentadéc-1-yne-5-one.

3. Procédé selon la revendication 1, caractérisé en ce que l'hydrazone de formule (II) est la 14-méthyl-bicyclo[10.3.0]pentadéc-[1(12)]-ène-13-one p-toluènesulfonylhydrazone et le composé cétonique acétylénique de formule (I) obtenu est la 3-méthyl-cyclopentadéc-1-yne-5-one.

4. Procédé selon l'une des revendications 1 à 3, caractérisé en ce que la réduction du réactif halogénant est effectuée au moyen d'un bisulfite de métal alcalin.

5. Procédé selon l'une des revendications 1 à 3, caractérisé en ce que le traitement de l'hydrazone de formule (II) au moyen du réactif halogénant s'effectue en présence d'un solvant auxiliaire appartenant à la classe des éthers ou en présence d'acétone.

**Claims**

1. Process for the preparation of acetylenic ketones of formula

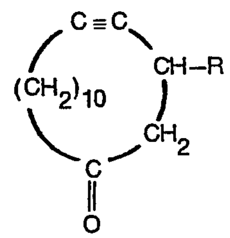

(I)

wherein symbol R represents an alkyl radical containing 1 to 3 carbon atoms or a hydrogen atom, characterized in that

a) a hydrazone of formula

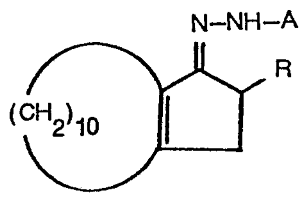

(II)

wherein symbol R is defined as indicated hereinabove and A designates a sulphonyl radical of formula

$$R^1SO_2— \qquad (III)$$

wherein $R^1$ represents an aryl radical, is treated with a halogenating reactant chosen among the following compounds:

a. bromine
b. chlorine
c. iodine
d. 1,3-dibromo-5,5-dimethyl-hydantoin
e. N-bromosuccinimide
f. N-iodosuccinimide and
g. N-chlorosuccinimide

in the presence of isopropyl alcohol, secondary butyl alcohol or ethylene-glycol and at a temperature of between −25° and +25°C;

b) the excess of halogenating reagent is reduced and

c) the reaction product is heated in an aqueous medium at a temperature of between 20° and 60°C.

2. Process according to claim 1, characterized in that the hydrazone of formula (II) is bicyclo[10.3.0]pentadec-[1(12)]-en-13-one-p-toluenesulphonylhydrazone and the obtained acetylenic ketone of formula (I) is cyclopentadec-1-yn-5-one.

3. Process according to claim 1, characterized in that the hydrazone of formula (II) is 14-methyl-bicyclo[10.3.0]-pentadec-[1(12)]-en-13-one p-toluenesulphonyl-hydrazone and the obtained acetylenic ketone of formula (I) is 3-methyl-cyclopentadec-1-yn-5-one.

4. Process according to any of claims 1 to 3, characterized in that the reduction of the halogenating reagent is effected by means of an alkali metal bisulfite.

5. Process according to any of claims 1 to 3, characterized in that the treatment of the hydrazone

of formula (II) with a halogenating reagent is effected in the presence of an auxiliary solvent belonging to the class of ethers or in the presence of acetone.

**Patentansprüche**

1. Verfahren zur Herstellung eines acetylenischen Ketons der Formel

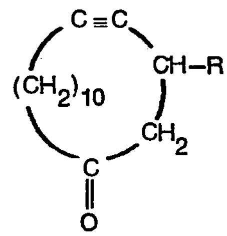

(I)

worin das Symbol R für eine Alkylgruppe mit 1 bis 3 Kohlenstoffatomen oder für ein Wasserstoffatom steht, dadurch gekennzeichnet, dass man

a) ein Hydrazon der Formel

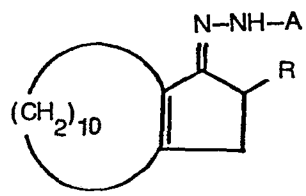

(II)

worin das Symbol R die oben genannte Bedeutung hat und A für eine Sulfonylgruppe der Formel

$$R^1SO_2—$$

(III)

in der $R^1$ eine Arylgruppe bedeutet, steht, mit einem Halogenierungsreagenz, das aus der nachstehenden Gruppe gewählt wird:

a. Brom
b. Chlor
c. Jod
d. 1,3-Dibromo-5,5-dimethyl-hydantoin
e. N-Bromsuccinimid
f. N-Jodsuccinimid und
g. N-Chlorsuccinimid;

in Anwesenheit von Isopropylalkohol, sekundärem Butylalkohol oder Aethylenglykol bei einer Temperatur von —25° bis +25°C umsetzt;

b) den Ueberschuss des Halogenierungsreagenzes reduziert und

c) das Reaktionsprodukt in einem wässrigen Milieu bei einer Temperatur von 20° bis 60°C erhitzt.

2. Verfahren gemäss Anspruch 1, dadurch gekennzeichnet, dass das Hydrazon der Formel (II) Bicyclo[10.3.0]pentadec-[1(12)]-en-13-on-p-toluolsulfonylhydrazon ist und das erhaltene acetylenische Keton der Formel (I) Cyclopentadec-1-in-5-on ist.

3. Verfahren gemäss Anspruch 1, dadurch gekennzeichnet, dass das Hydrazon der Formel (II) 14-Methyl-bicyclo[10.3.0]pentadec-[1(12)]-en-13-on-p-toluolsulfonyl-hydrazon ist und das erhaltene acetylenische Keton der Formel (I) 3-Methyl-cyclopentadec-1-in-5-on ist.

4. Verfahren gemäss einem der Ansprüche 1 bis 3, dadurch gekennzeichnet, dass die Reduktion des Halogenierungsreagenzes mittels eines Alkalimetalbisulfits durchgeführt wird.

5. Verfahren gemäss einem der Ansprüche 1 bis 3, dadurch gekennzeichnet, dass die Umsetzung des Hydrazons der Formel (II) mittels eines Halgenierungsreagenzes in Anwesenheit eines zusätzlichen Lösungsmittels aus der Gruppe der Aether oder von Aceton durchgeführt wird.